**Europäisches Patentamt**

**European Patent Office**

**Office européen des brevets**

(19)

(11) Veröffentlichungsnummer : **0 258 558 B2**

(12)
# NEUE EUROPÄISCHE PATENTSCHRIFT

(45) Veröffentlichungstag der neuen Patentschrift :
**26.01.94 Patentblatt 94/04**

(51) Int. Cl.$^5$ : **A61K 7/00,** A61K 9/10,
B01F 17/00, B01F 17/54

(21) Anmeldenummer : **87109186.4**

(22) Anmeldetag : **26.06.87**

(54) **Kosmetisches Mittel, insbesondere feuchtigkeitsbindendes Hautpflegemittel.**

Die Akte enthält technische Angaben, die nach dem Eingang der Anmeldung eingereicht wurden und die nicht in dieser Patentschrift enthalten sind.

(30) Priorität : **12.08.86 DE 3627313**

(43) Veröffentlichungstag der Anmeldung :
**09.03.88 Patentblatt 88/10**

(45) Bekanntmachung des Hinweises auf die Patenterteilung :
**24.04.91 Patentblatt 91/17**

(45) Bekanntmachung des Hinweises auf die Entscheidung über den Einspruch :
**26.01.94 Patentblatt 94/04**

(84) Benannte Vertragsstaaten :
**AT BE CH DE ES FR GB GR IT LI LU NL SE**

(56) Entgegenhaltungen :
**DE-A- 2 163 541**
**DE-A- 2 905 257**
**DE-A- 3 110 258**
**H. JANISTYN: "Handbuch der Kosmetika und Riechstoffe", Band 3: "Die Körperpflegemittel", 1973, Seiten 604-611, A. HÜTHIG, Heidelberg**
**E.W. FLICK: "Cosmetic & Toiletry formulations", 1984, Seiten 379,384,518,542, Noyes Publications, Pard Ridge, New Jersey, US**

(73) Patentinhaber : **Fribad Cosmetics GmbH**
**Ruhrstr. 7**
**D-76532 Baden-Baden (DE)**

(72) Erfinder : **Feger, Marlene, Dr.**
**Lindenstrasse 22**
**D-7583 Ottersweier (DE)**

(74) Vertreter : **Pfenning, Meinig & Partner**
**Mozartstrasse 17**
**D-80336 München (DE)**

EP 0 258 558 B2

EP 0 258 558 B2

## Beschreibung

Die Erfindung betrifft ein feuchtigkeitsbindendes Hautpflegemittel, auf der Basis einer O/W (Öl in Wasser)-Emulsion mit feuchtigkeitsbindenden Wirkstoffen, einem Emulgator mit überwiegend hydrophilen Gruppen und weiteren Zusätzen.

Als flüssige Hauptpflegemittel werden je nach Zielrichtung O/W-Emulsionen oder W/O-Emulsionen verwendet. O/W-Emulsionen lassen sich leicht auftragen, werden vom Benutzer als angenehm empfunden und geben keinen Fettglanz. Die Schutzwirkung jedoch ist schlecht, insbesondere die Feuchtigkeitsbindung. Bei diesen Mitteln wird im Gegenteil an der Haut ein austrocknender Effekt beobachtet. Demgegenüber verleihen W/O-Emulsionen eine gute Schutz- und Pflegewirkung, doch wird die fettige und klebrige Schicht vom Benutzer als unangenehm empfunden (G.A. Nowak "Die kosmetischen Präparate" 3. Auflage, Band 2, S. 216, 217).

Alle kosmetischen Mittel in Form einer O/W- oder W/O-Emulsion enthalten eine amphiphile Verbindung oder einen sogenannten Emulgator, der die Grenzflächenspannung zwischen den beiden Hauptkomponenten Öl und Wasser herabsetzt und die Bildung einer Emulsion ermöglicht. Je nach Art der Emulsion überwiegen die hydrophilen Gruppen (O/W-Emulsion) oder die lipophilen Gruppen (W/O-Emulsion). Da es sich bei Emulsionen um chemisch-physikalisch instabile Systeme handelt, die zur Trennung in ihre ölige und wässrige Phase neigen, sind zusätzlich zu den eingangs genannten Komponenten Stabilisatoren, z.B. Wachse, Salze, Verdickungsmittel etc, notwendig. Mit Hilfe dieser Stabilisatoren kann die Emulsion über einen längeren Zeitraum stabil gehalten werden (Janistyn "Handbuch der Kosmetika und Riechstoffe" 2. Auflage, Band III, S. 605 bis 610 und Flick "Cosmetic and Toiletry Formulations" Noyes Publcations 1984, S. 379, 384, 518, 542).

Wie bereits zuvor angedeutet, läßt sich eine effektive feuchtigkeitsbildende und pflegende Wirkung nur mit W/O-Emulsionen erzielen, doch werden diese Präparate als nicht so angenehm empfunden wie die O/W-Emulsionen. Der Erfindung liegt nun die Aufgabe zugrunde, die Vorteile der W/O-Emulsion hinsichtlich der Auftragsfähigkeit und des individuellen Empfindens des Benutzers mit den Vorteilen der W/O-Emulsion hinsichtlich der Schutz- und Pflegewirkung zu kombinieren und dabei auf belastende Hilfsstoffe weitgehend zu verzichten.

Diese Aufgabe wird bei dem eingangs genannten Hautpflegemittel dadurch gelöst, daß das Mittel frei von Emulsions stabilisatoren ist und der Emulgator mit einem Anteil von $\leq$ 1% zugegeben wird, und daß aus einer zunächst in getrennten Phasen vorliegenden, fette Öle, mineralische Öle oder ätherische Öle enthaltenden O/W-Emulsion und einer wässrigen Phase die aufzutragende instabilen O/W-Emulsion unmittelbar vor Anwendung des Mittels durch Schütteln hergestellt wird.

Durch den Verzicht auf Emulsions stabilisatoren entfallen die bisher üblichen Ballaststoffe ohne kosmetische Wirkung. Dies führt dazu, daß die Emulsion nicht stabil ist, d.h. nach relativ kurzer Zeit eine Phasentrennung eintritt. Der Anteil des Emulgators, der üblicherweise zwischen 2 und 12% beträgt, ist bei der erfindungsgemäßen Zusammensetzung erheblich herabgesetzt. Dieser geringe Anteil führt einerseits dazu, daß nach der Trennung eine Phase in Form einer O/W-Emulsion vorliegt, die sich auf der anderen Phase einer wässrigen Phase, absetzt. Andererseits reicht dieser geringe Anteil des Emulgators aus, um durch Schütteln die gesamte Menge zu emulgieren und damit auftragsfähig zu machen. Dabei wurde beobachtet, daß dieses Mittel hinsichtlich der Auftragsfähigkeit und des individuellen Empfindens des Benutzers - wie an sich zu erwarten - einer O/W-Emulsion entspricht, während zugleich aber die feuchtigkeitsbindende und pflegende Wirkung einer W/O-Emulsion erreicht wird. Dies dürfte in erster Linie auf den Wegfall der hautbelastenden Stabilisatoren zurückzuführen sein.

Praktische Untersuchungen haben ein erheblich verbessertes Feuchtigkeits-Bindevermögen als bei einer herkömmlichen O/W-Emulsion gezeigt. Zum Vergleich wurden eine O/W-Emulsion mit herkömmlichen Stabilisatoren und einem Emulgator-Anteil von 1,5% mit den gleichen feuchtigkeitsbindenden Wirkstoffen ausgestattet, wie die erfindungsgemäße Emulsion mit einem Emulgator-Anteil von nur 0,05% und ohne Stabilisatoren. Während die herkömmliche O/W-Emulsion noch eine austrocknenden Effekt zeigte, was durch Messungen an der Haut belegbar ist, ergab sich bei dem erfindungsgemäßen Mittel eine hohe wasserretinierende Wirkung.

Die exakte Menge des Emulgators richtet sich nach dessen HLB-Wert (Hydrophile Lipophile Balance) und nach dem Mischungsverhältnis von wässriger und öliger Phase. Eine Rahmen-Rezeptur des erfindungsgemäßen Mittels weist folgende Zusammensetzung auf :

ca. 10-90% lipophile Bestandteile
ca. 90-10% hydrophile Bestandteile
ca. 0,01 bis 0,5% Emulgator.

Bei dem erfindungsgemäßen Mittel kann die wässrige Phase neben Wasser, insbesondere wasserlösliche Wirkstoffe, Glykole, Alkohole, Konservierungs- und gegebenenfalls Farbstoffe enthalten, während die emulgierte Phase zusätzlich öllösliche Wirkstoffe, Konservierungsstoffe und gegebenenfalls Parfüm, enthält.

2

Gemäß einem Ausführungsbeispiel weist das Mittel gemäß der Erfindung folgende Zusammensetzung auf:

Wasserphase:

| | |
|---|---|
| Aqua demin. | 39,25 % |
| Hyaluronsäure | 0,10 % |
| Mannuronsäuresilanol | 20,00 % |
| Pflanzenextrakt | 13,00 % |
| D-Panthenol | 2,00 % |

Ölphase:

| | |
|---|---|
| Avocadoöl | 20,00 % |
| Vitamin-E-Acetat | 5,00 % |
| Parfüm | 0,25 % |
| Konservierungsstoff | 0,30 % |

Emulgator:

| | |
|---|---|
| Trilaneth-4-Phosphate | 0,10 % |
| | 100,00 %. |

Werden dem erfindungsgemäß zusammengesetzten Mittel Substanzen mit erhöhtem Lichtschutzfaktor zugesetzt, so läßt sich das erfindungsgemäße Mittel als Sonnenschutzmittel verwenden. Diese Verwendung hat überraschenderweise gezeigt, daß das Mittel auch von solchen Personen, die gegenüber herkömmlichem Sonnenschutzmittel allergisch reagieren, ohne Unverträglichkeitsreaktionen benutzt werden kann. Für die Auslösung solcher Allergien kommen vornehmlich die in herkömmlichen Sonnenschutzmitteln verwendeten Emulgatoren in Frage. Möglicherweise sind diese, wie auch die üblicherweise verwendeten Stabilisatoren, ferner auslösende Faktoren für zeitweilig beobachtete Unverträglichkeitsreaktionen bei feuchtigkeitsbindenden Hautpflegemitteln.

**Patentansprüche**

1.  Feuchtigkeitsbindendes Hautpflegemittel auf der Basis einer O/W (Öl-in-Wasser)-Emulsion mit feuchtigkeitsbindenden Wirkstoffen, einem Emulgator mit überwiegend hydrophilen Gruppen und weiteren Zusätzen, dadurch gekennzeichnet, daß das Mittel frei von Emulsionsstabilisatoren ist, der Emulgator mit einem Anteil $\leqq 1$ % zugegeben wird und das Mittel aus einer fette Öle, miteralische Öle oder ätherische Öle enthaltenden O/W-Emulsion und einer wässrigen Phase besteht, dessen Auftrag auf die Haut durch Schütteln des Mittels unmittelbar vor seiner Anwendung unter Bildung einer istabilen O/W-Emulsion erfolgt.

2.  Mittel nach Anspruch 1, gekennzeichnet durch
    10-90% lipophile Bestandteile;
    90-10% hydrophile Bestandteile;
    0,01-0,5% Emulgator.

3.  Mittel nach Anspruch 1 und 2, dadurch gekennzeichnet, daß die wässrige Phase neben Wasser wasserlösliche Wirkstoffe, Glykole, Alkohole, Konservierungsmittel und gegebenenfalls Farbstoffe enthält.

4.  Mittel nach einem der Ansprüche 1 bis 3, dadurch gekennzeichnet, daß die emulgierte Phase zusätzlich öllösliche Wirkstoffe, Konservierungsstoffe und gegebenenfalls Parfüm enthält.

5. Mittel nach einem der Ansprüche 1 bis 4, gekennzeichnet durch folgende Zusammensetzung:

| | |
|---|---|
| **Wasserphase** | |
| Aqua demin. | 39,25 % |
| Hyaluronsäure | 0,10 % |
| Mannuronsäuresilanol | 20,00 % |
| Pflanzenextrakt | 13,00 % |
| D-Phanthenol | 2,00 % |
| | |
| **Ölphase** | |
| Avocadoöl | 20,00 % |
| Vitamin-E-Acetat | 5,00 % |
| Parfüm | 0,25 % |
| Konservierungsstoff | 0,30 % |
| | |
| **Emulgator** | |
| Trilaneth-4-Phosphate | 0,10 % |
| | 100,00 %. |

6. Verwendung des Mittels gemäß einem der Ansprüche 1 bis 5 als Sonnenschutzmittel durch Zugabe eines Lichtschutzmittels.

**Claims**

1. Skin hydrating product based on an O/W (oil-in-water) emulsion with hydrating active agents, an emulsifier with mainly hydrophilic groups and further additives, characterized in that the product is free of emulsion stabilisers, that the emulsifier is added in a proportion of $\leq$ 1 % and that the product consists of an O/W emulsion containing fatty oils, mineral oils or essential oils and of an aqueous phase, its application to the skin being effected by shaking the product immediately before use, an unstable O/W emulsion being formed.

2. Product according to claim 1, characterized by 10 to 90% of lipophilic constituents, 90 to 10% of hydrophilic constituents and 0.01 to 0.5% emulsifier.

3. Product according to claims 1 and 2, characterized in that, apart from water, the aqueous phase contains water-soluble active agents, glycols, alcohols, preservatives and optionally dyes.

4. Product according to one of the claims 1 to 3, characterized in that the emulsified phase additionally contains oil-soluble active agents, preservatives and optionally perfume.

5. Product according to one of the claims 1 to 4, characterized by the following composition:

| aqueous phase | |
|---|---|
| demineralized water | 39.25% |
| hyaluronic acid | 0.10% |
| mannuronic acid silanol | 20.00% |
| Plant extract | 13.00% |
| D-phanthenol | 2.00% |
| | |
| oil phase | |
| avocado oil | 20.00% |
| vitamin-E acetate | 5.00% |
| perfume | 0.25% |
| preservative | 0.30% |
| | |
| emulsifier | |
| trilaneth-4-phosphates | 0.10% |
| | 100.00% |

6. Use of the product according to one of the claims 1 to 5 as a sun screening product by the addition of a light protecting agent.

**Revendications**

1. Produit d'hydratation de la peau à base d'une émulsion H/E (huile dans l'eau) contenant des substances actives hydratantes, un émulsionnant à groupes principalement hydrophiles, ainsi que d'autres additifs, caractérisé en ce que le produit est exempt de stabilisateurs d'émulsions, en ce que l'émulsionnant est ajouté en proportion ≦1 % et en ce que le produit est composé d'une émulsion H/E contenant des huiles grasses, des huiles minérales ou des huiles éthérifiées, et d'une phase aqueuse, dont l'application sur la peau s'effectue par agitation du produit immédiatement avant son utilisation en formant une émulsion H/E instable.

2. Produit selon la revendication 1, caractérisé par :
   10 - 90% de composants lipophiles ;
   90 - 10% de composants hydrophiles ;
   0,01 - 0,5% d'émulsionnant.

3. Produit selon les revendications 1 et 2, caractérisé en ce que la phase aqueuse contient, outre de l'eau, des substances actives hydrosolubles, des glycols, des alcools, des agents de conservation et éventuellement des colorants.

4. Produit selon l'une des revendications 1 à 3, caractérisé en ce que la phase émulsionnée contient en outre des substances actives solubles dans l'huile, des agent de conservation et éventuellement un parfum.

5. Produit selon l'une des revendications 1 à 4, caractérisé par la composition suivante :

```
Phase aqueuse
        eau déminéralisée              39,25%
        acide hyaluronique              0,10%
        mannuronate de silanol         20,00%


        extrait de plantes             13,00%
        D - panthénol                   2,00%
Phase huileuse
        huile d'avocat                 20,00%
        acétate de vitamine E           5,00%
        parfum                          0,25%
        agent de conservation           0,30%
Emulsionnant
        trilaneth-4-phosphate           0,10%
                                       ─────────
                                       100,00%
```

6. Utilisation du produit selon l'une des revendications 1 à 5 comme produit antisolaire par addition d'un produit de protection contre la lumière.